# EUROPÄISCHE PATENTSCHRIFT

(11) **EP 1 411 837 B1**
(45) Veröffentlichungstag und Bekanntmachung des Hinweises auf die Patenterteilung: **13.10.2010**
(21) Anmeldenummer: 02794533.6
(22) Anmeldetag: 01.08.2002
(51) Int. Cl.: A61B 17/04

(54) **CHIRURGISCHES INSTRUMENT ZUM PLATZIEREN EINES HARNINKONTINENZBANDES IM UNTERLEIB VON PATIENTEN**
SURGICAL INSTRUMENT FOR PLACING A URINARY INCONTINENCE PAD IN THE LOWER ABDOMEN OF PATIENTS
INSTRUMENT CHIRURGICAL PERMETTANT DE PLACER UNE BANDE D'INCONTINENCE URINAIRE DANS LE BAS-VENTRE DE PATIENTS

(30) Priorität: 03.08.2001 DE 10138955
(43) Veröffentlichungstag der Anmeldung: 28.04.2004
(73) Patentinhaber: Aesculap AG, 78532 Tuttlingen (DE)
(72) Erfinder: ODERMATT, Erich, K., CH-8200 Schaffhaussen (CH); WEIS, Christine, 78532 Tuttlingen (DE); WÖLFLE, Werner, 78073 Bad Dürrheim (DE); MELCHIOR, Hansjörg, 34117 Kassel (DE); WEISSHAUPT, Dieter, 78194 Immendingen (DE); TADDIA, Lino, 78532 Tuttlingen (DE)
(74) Vertreter: Patentanwälte Ruff, Wilhelm, Beier, Dauster & Partner
(86) Internationale Anmeldenummer: PCT/EP2002/008574
(87) Internationale Veröffentlichungsnummer: WO 2003/013369

(56) Entgegenhaltungen:
- EP-A- 1 093 758
- WO-A-00/74633
- WO-A-01/52750
- US-A- 5 899 909

## Beschreibung

Die Erfindung betrifft ein chirurgisches Instrument zum Platzieren eines Harninkontinenzbandes im Unterleib von Patienten, insbesondere weiblichen Patienten, gemäß des Oberbegriffes des Anspruchs 1. Ein derartiges Instrument ist aus der WO-A- 00/74633 bekannt.

Zur Behebung der Harninkontinenz sind verschiedene Operationsmethoden und speziell dafür ausgebildete Operationsbestecke und Implantate entwickelt worden. Einige Operationsmethoden erfordern die Öffnung des Bauchraumes, um prothesenartige Gegenstände einzubringen, die die Harnröhre im Bereich des Blasenhalses umschließen oder sie von oben her drücken oder von unten her anheben (WO 00/18319; WO 90/01016; WO 91/00069; US 4,709,690; WO 85/02993). Besonders bewährt hat sich die minimalinvasive Einbringung eines Inkontinenzbandes, das beispielsweise bei weiblichen Patienten durch die Vaginalwand hindurchgeführt wird und als U-förmige Schlinge, die die Harnröhre untergreift, im Bauchraum platziert wird, wobei die freien Enden der Schlinge entweder in der Bauchdecke befestigt, beispielsweise vernäht werden oder in der Bauchdecke frei enden und dort durch einwachsendes Bindegewebe verankert werden. Chirurgische Instrumente und solche Bänder sind in den WO 90/03766, WO 96/06567, WO 97/13465 und WO 2001/030246 beschrieben.

Gegenstand der Erfindung ist ein chirurgisches Instrument zum Platzieren eines Harninkontinenzbandes im Unterleib von Patienten, insbesondere weiblichen Patienten, mit mindestens einem gebogenem Schaft zum Durchdringen des Unterleibes, einem dem Schaft zugeordneten Handgriff für den Schaft und mindestens einer Befestigungseinrichtung für das Band am Schaft. Das Bandende ist mittels der Befestigungseinrichtung mit dem Schaft unlösbar zu verbinden, wobei das Bandende beim Trennen des Bandes vom Schaft an diesem untrennbar verbleibt. Der Schaft kann nadelförmig ausgebildet sein und einen runden Querschnitt aufweisen. Es ist aber auch möglich, den Schaft mit einer von der Kreisform abweichenden Querschnittsform auszubilden, insbesondere mit einer flachen oder sich über die Schaftlänge ändernden Querschnittsform, worauf später noch eingegangen wird. Der Schaft kann in verschiedener Länge zur Anpassung an die Größe und Beleibtheit des Patienten vorgesehen sein. In der Regel ist der Schaft zur lösbaren Verbindung mit dem Griff ausgebildet, insbesondere axial und drehgesichert am Schaft festlegbar. Dies ermöglicht die Befestigung unterschiedlicher Schäfte am Griff und auch die Mehrfachverwendung des Griffes bei zur Einmalverwendung vorgesehenen Schäften. Der Schaft kann gleichmäßig oder symmetrisch gekrümmt sein. Es ist jedoch vorzugsweise vorzusehen, daß der Schaft asymmetrisch gekrümmt ist, wodurch ein sicheres Einführen des Schaftes zwischen Blase und Schambein möglich ist. Dabei kann die Krümmung des Schaftes in Abhängigkeit davon ausgebildet sein, ob der Schaft vom Bauchraum aus in Richtung Harnröhre eingeführt wird oder in umgekehrter Richtung. Erfindungsgemäß ist vorzugsweise der Schaft derart asymmetrisch gekrümmt, daß er alternativ von der Bauchhöhle aus oder vom Schritt des Patienten aus in den Unterleib einfügbar ist.

Der Schaft kann an beiden Enden Einführspitzen zum Durchdringen des Unterleibes aufweisen oder mit solchen verbindbar sein. Dies ermöglicht es dem Operateur, das eine oder andere Ende zum Einführen in den Körper auszuwählen. Vorzugsweise ist der Schaft an beiden Enden zur lösbaren Verbindung mit dem Griff ausgebildet. Dies ermöglicht einerseits die alternative beidseitige Verwendung des Schaftes. Auf der anderen Seite ist es auch möglich nach Einführung des Schaftes in den Unterleib den Griff mit der aus dem Körper herausschauenden Spitze des Schaftes, gegebenenfalls nach Abnehmen einer lösbaren Spitze, mit dem Schaft zu verbinden und den Schaft mit Hilfe des Griffes aus dem Körper unter gleichzeitiger Einführung des Bandes herauszuziehen. Vorzugsweise ist auch der Schaft an beiden Enden zur Verbindung mit dem Band ausgebildet, wodurch sich vielfältige Alternativmöglichkeiten bei der Operation ergeben.

Bei einer Ausführungsform der Erfindung ist die Befestigungseinrichtung zur Befestigung des Bandes am Schaft so ausgebildet und platziert, daß das Band erst nach Entfernung des Griffs vom Schaft an diesem befestigbar ist. Dadurch wird zwangsweise erreicht, daß das Band erst dann am Schaft befestigt werden kann, wenn dieser bereits in den Körper eingeführt ist und der Griff gelöst wird. Dadurch wird vermieden, daß das Band beim Einführen des Schaftes in den Körper kontaminiert wird. Es kann auch atraumatisch und schonend platziert werden. Die Befestigungseinrichtungen am Schaft für Griff und Band können die gleichen sein. Sie können aber auch verschieden sein und im wesentlichen an der gleichen Stelle vorgesehen sein, so daß sich die alternativen Befestigungsmöglichkeiten gegenseitig ausschließen.

Bei einer weiteren Ausführungsform ist, wie oben bereits erwähnt, aus hygienischen Gründen vorgesehen, daß der Schaft nur ein Mal verwendbar ist und dann verworfen wird. Unabhängig davon ist es aber in der Regel zweckmäßig und erwünscht, daß jedem Bandende ein eigener Schaft zugeordnet ist, also eine zweimalige Verwendung des selben Schaftes für die beiden Bandenden bei einer Operation ausgeschlossen ist. Hierzu ist der Schaft vorzugsweise mit einer Sicherungseinrichtung ausgerüstet, die eine Wiederverwendung des Schaftes zumindest während einer Operation ausschließt.

Das Bandende kann unmittelbar am Schaft befestigbar sein, so kann das Bandende durch eine Öse am Schaft hindurchführbar sein oder das Schaftende kann als Fangnadel oder Karabiner ausgebildet sein und ein schlingenförmig ausgebildetes oder gelochtes Bandende ergreifen. Vorzugsweise ist das Band mittels eines am Bandende befestigten Kupplungsstückes am Schaft befestigbar, wobei insbesondere Schaftende und Kupplungsstück als Steckverbindung ausgebildet sind. Zur Verhinderung der Wiederverwendung des Schaftes ist das Bandende und insbesondere das am Bandende befestigte Kupplungsstück unlösbar mit dem Schaft zu verbinden. Nach Einführen des Bandes in den Unterleib kann der überstehende Teil des Bandes abgeschnitten werden, wobei das Bandende dann am Schaft verbleibt. Das Band kann mit Hilfe einer selbstsperrenden Rastverbindung am Schaft befestigbar sein, entweder unmittelbar oder über das Kupplungsstück. Ist die Rastverbindung unlösbar oder nur mit Hilfe eines externen Werkzeuges lösbar, dann ist eine Wiederverwendung zumindest während der Operation unmöglich gemacht.

Die Spitze des Schaftes ist vorzugsweise so ausgebildet, dass sie nicht selbstschneidend, insbesondere stumpf ist. Dadurch werden Verletzungen der Bauchorgane vermieden. Die Ein- und Ausführstellen des Schaftes im Körper können mit dafür vorgesehenen Instrumenten, beispielsweise einem Skalpell, geöffnet werden. Der Schaft kann unter Fingerführung durch den Körper geführt werden, indem ein Finger des Operateurs den Schaft durch Ertasten und Herausführen abholt.

Der Schaft ist vorzugsweise in den Griff einsteckbar ausgebildet. Der Griff kann zur Axialverriegelung des Schaftes in Ausnehmung des Schaftes vorzugsweise federnd eingreifbare Rastglieder aufweisen, die in Raststellung verriegelbar sind. Die Rastglieder können mit Hilfe einer lösbaren Sperre, insbesondere mit Hilfe eines Sperrschiebers des Griffs verriegelbar sein, wobei der Sperrschieber vorzugsweise zumindest in Schließstellung, insbesondere in seinen beiden Endstellungen kraftschlüssig gehalten ist. Bei dieser Ausführungsform kann das Schaftende an den federnden Rastgliedern vorbei eingesteckt und herausgezogen werden und in der Einsteckstellung verriegelt werden. Bei einer anderen Ausführungsform der Erfindung sind die Rastglieder des Griffs selbstsperrend ausgebildet und wirken mit einem Entsperrglied des Schiebers zusammen, das vorzugsweise durch Federkraft in Sperrlage gehalten ist. Bei dieser Ausführungsform sperren die Rastglieder automatisch nach Einführen des Schaftes, so daß sich eine besondere Verriegelung erübrigt. Erst nach Entriegelung ist der Schaft wieder herausziehbar. Es ist auch möglich, den Schaft am Griff axial zu verspannen, insbesondere mittels einer axialen Verschraubung. Der Schaft kann an seinem Einsteckende in den Griff mit einem Gewinde, insbesondere einem Außengewinde, versehen sein, wobei ein Schraubglied im Griff ein entsprechendes Gegengewinde aufweist.

Eine Verdrehsicherung des Schaftes im Griff wird vorzugsweise dadurch erreicht, daß der Schaft im Bereich des in den Griff einführbaren Schaftendes einen von der Kreisform abweichenden Querschnitt, insbesondere einen abgeflachten oder eckigen Querschnitt besitzt. Entsprechend ausgebildete und anliegende Flächen des Griffes verhindern eine Drehung. Weiterhin kann durch eine geeignete Passung und eine ausreichende gegenseitige Verbindungslänge eine kippfreie und damit wackelfeste Verbindung des Schaftes im Griff erreicht werden.

Bei einer Ausführungsform der Erfindung ist der Schaft als Hohlkörper ausgebildet, insbesondere als Rohr, der zumindest nach den Seiten geschlossen ist. Dies ermöglicht es, das Band innerhalb des Schaftes anzuordnen, so daß der Schaft während des Einführens in den Unterleib als Schutzhülle für das Band dient. Der Schaft kann an einem Ende, insbesondere an dem von der Einführrichtung abweisenden Ende offen sein, so daß das Band festgehalten werden kann, während der Schaft aus dem Körper herausgezogen wird.

In der Regel ist der Schaft massiv ausgebildet, was eine kostengünstige Herstellung ermöglicht und es auch erlaubt, den Schaft mit geringem Querschnitt auszubilden und dadurch auch den Stichkanal im Körper im Querschnitt möglichst klein zu halten. Der Schaft kann wie üblich, einen kreisrunden Querschnitt besitzen. Bei besonderen Ausführungsformen, besitzt der Schaft jedoch einen von der Kreisform abweichenden Querschnitt. Dadurch kann insbesondere der flächenhaften Ausdehnung des Bandes Rechnung getragen werden. So ist bei einer Ausführungsform der Erfindung der Schaft mindestens in einem Abschnitt, vorzugsweise über seine gesamte Länge als Flachform ausgebildet. Die Breite des Schaftes kann zumindest in den verbreiterten Abschnitten, insbesondere über seine gesamte Länge im wesentlichen der Breite des Bandes entsprechen. Dadurch kann, falls erforderlich aufgrund der nicht scharfen Längsränder des Schaftes eine stumpfe Verbreiterung des Kanales durch das Gewebe erreicht werden, was wiederum eine vorbestimmte orientierte Lage des Bandes im Körper gewährleistet. Auf diese Weise kann ein Verdrehen oder Einrollen des Bandes beim Einführen in den Körper vermieden werden. Der Schaft kann im Querschnitt flach, oval oder eliptisch ausgebildet sein. Es ist auch möglich, einen Stab mit kreisrundem Querschnitt durch seitliche Flügel zu verbreitern. Es ist auch möglich den Stab mindestens abschnittsweise breiter auszubilden als die Breite des Bandes, was insbesondere bei adipösen Patienten günstig ist. Übergänge in der Breite verlaufen vorzugsweise allmählich. Die Flachform liegt bei einer Ausführungsform senkrecht zur Krümmungsebene. Bei einer anderen Ausführungsform liegt sie in der Krümmungsebene des Schaftes.

Das Band ist mit Vorteil verdrehsicher am Schaft befestigbar. Dadurch kann die Orientierung des Bandes im Körper vorgegeben werden. Vorzugsweise ist das Band relativ zum Schaft so fixierbar, daß die Bandebene in der Biegungsebene und/oder der Ebene einer Flachseite des Schaftes liegt. Dadurch kann erreicht werden, daß das Band flach unterhalb der Harnröhre zur Anordnung kommt und ohne Verdrehung bis zur Bauchwand geführt werden kann. Auch bei einer im Querschnitt runden Nadel kann die flächige Ausrichtung des Bandes vorgegeben werden. So kann das Bandende im Bereich des Schaftendes im Querschnitt V- oder U-förmig vorgebogen sein, so daß es im weiteren Verlauf von selbst die Flachform annimmt. Es kann auch einmal gefaltet oder zickzack-förmig mehrfach gefaltet sein, wodurch es von selbst im weiteren Verlauf die Flachform annimmt.

Schaft und Griff können aus Edelstahl bestehen. Es ist aber auch möglich, den Griff aus Kunststoff zu fertigen, was bevorzugt ist. Auch der Schaft selbst kann ganz oder teilweise aus Kunststoff bestehen.

Bei einer anderen Ausführungsform der Erfindung sind Schaft und Griff unlösbar miteinander verbunden, insbesondere einstückig ausgebildet. Insbesondere bei dieser Ausführungsform ist die Befestigungseinrichtung für das Band am freien Schaftende im Bereich der Spitze vorgesehen. Bei dieser Ausführungsform kann das Band beim Einführen des Schaftes mitgezogen werden. Es ist auch möglich den Schaft von einer Seite her durchzustechen, dann am herausschauenden Schaftende das Band zu befestigen und mit dem Schaft rückwärts mitzuziehen. Die Ausführungsform mit fester Verbindung zwischen Schaft und Griff ist besonders dort von Vorteil, wo auf eine Wiederverwendbarkeit großer Wert gelegt wird. Eine solche Ausführungsform kann mit einer leicht zu reinigenden Oberfläche ausgebildet werden, indem Hinterschneidungen und sonstige schwer zugängliche Stellen, an denen sich Schmutz ablagern kann, vermieden werden.

Weiterhin kann es gemäß der Erfindung vorgesehen sein, den Schaft mit einer flexiblen Hülle zu umgeben, aus der der Schaft, insbesondere beim Durchziehen des Bandes, herausziehbar ist. Eine solche flexible Hülle kann aus resorbierbarem Material bestehen und zum vorübergehenden Verbleib im Körper vorgesehen sein. Weiterhin kann die flexible Hülle zur Befestigung und/oder Aufnahme des Bandes dienen oder auch selbst als Inkontinenzband ausgebildet sein.

Die Fixierung des Bandes im Körper kann durch Annähen erfolgen. In der Regel reichen strukturierte Oberflächen und/oder Randabschnitte für die Selbstfixierung aus. Das Inkontinenzband selbst ist flexibel ausgebildet. Es kann ein textiles Band sein, insbesondere ein solches, das beschichtet oder imprägniert ist. Es kann auch ein mindestens abschnittsweise glattes Band in Form einer Folie sein. Es kann mit Vorteil, in Längsrichtung gesehen, in Material und/oder Struktur verschieden ausgebildet sein. Dies ist besonders für den mittleren Längsabschnitt von Bedeutung, der zur Anordnung im Bereich der Harnröhre bestimmt ist. Die Harnröhre ist sehr empfindlich gegenüber Reizungen, so daß in diesem Bereich das Band möglichst eine glatte Oberfläche aufweist. Das Band kann weiterhin mindestens teilweise aus resorbierbarem Material bestehen, so kann der mittlere Längsabschnitt, der zur Anordnung im Bereich der Harnröhre bestimmt ist, aus resorbierbarem Kunststoff bestehen. So ist es möglich, durch Wahl des resorbierbaren Materials und/oder der Struktur des Bandes zum Beispiel durch Poren, an dieser Stelle eine Vermehrung von Bindegewebe hervorzurufen, das die gewünschte Anhebung der Harnröhre. Dadurch hat das Inkontinenzband seine Aufgabe erfüllt und ist nicht mehr von Nöten. Die restlichen Abschnitte des Bandes können aus nicht resorbierbarem Material bestehen und im Körper verbleiben. Die resorbierbaren Materialien sind vorzugsweise nicht biologischen Ursprungs um Infektionen und immunotogische Abwehrreaktionen zu vermeiden. Es eignen sich die bekannten Polymere und Copolymere von Lactid, Glykolid, Trimethylencarbonat, Dioxanon und ε-Caprolacton. Besonders geeignet ist auch Polyvinylalkohol, dessen Löslichkeit und Resorptionsgeschwindigkeit durch chemische Modifizierung und/oder physikalische Behandlung (Kristallitbildung) beeinflusst werden können.

Das Band kann auch mit mehreren im wesentlichen parallelen Längsstreifen mit verschiedener Resorbierbarkeit ausgebildet sein, mit dem Ziel, Fremdmaterial zu entfernen sobald es nicht mehr benötigt wird. Unterschiede, insbesondere in Längsrichtung, insbesondere in Bezug auf den mittleren Längsabschnitt, können sich auch auf die mechanische Ausbildung beziehen. So können dort die Längskanten des Bandes mit besonderem Vorteil glatt ausgebildet sein wie vorzugsweise der gesamte Längsabschnitt. Es kann in diesem Längsabschnitt auch eine geschlossene insbesondere glatte Oberfläche vorgesehen sein. Es kann eine größere Breite und/oder größere Dicke vorgesehen sein als in übrigen Bereichen.

Bei einer besonderen Ausführungsform der Erfindung ist die Dicke des Bandes insbesondere in seinem mittleren Bereich veränderbar. Das Band kann insbesondere im mittleren Längsabschnitt doppelwandig, vorzugsweise schlauchförmig ausgebildet sein. Es kann insbesondere im mittleren Längsabschnitt, eine mit einem Fluid füllbare Kammer aufweisen, Diese kann je nach Füllgrad in Dicke und/oder Breite veränderbar sein. Hierzu kann die Kammer mindestens teilweise aus elastisch dehnbarem Material bestehen. Die Wandungen der mit dem Fluid füllbaren Kammer können mindestens teilweise aus durchstechbarem und selbst abdichtendem Material bestehen, insbesondere Silikonkautschuk. Dadurch kann die Kammer nachträglich durch Befüllung oder Absaugen in Größe und Form verändert werden. Es ist auch möglich, die mit Fluid füllbare Kammer mit einer zumindest zu einem Bandende führenden Fluidleitung zu versehen. Die Ausbildung einer solchen Kammer und einer damit verbundenen Fluidleitung kann bei einem thermoplastischen Material beispielsweise durch Verschweißung der Schlauchwandungen mit gewünschter Linienführung vorgenommen werden. Das Band besitzt mit Vorteil in den Bereichen, die zur Anordnung in der Bauchdecke bestimmt sind, eine die Selbstverankerung in der Bauchdecke begünstigende aufgelockerte Oberfläche und/oder poröse Struktur. Zweckmäßigerweise sind beide Bandenden zur Befestigung an einem zur Platzierung im Bauchraum dienenden, insbesondere gebogenem, Schaft ausgebildet. Hierzu können die Bandenden versteift sein. Die Bandenden können schmaler sein als das übrige Band. Ferner kann an den Bandenden eine Lochung vorgesehen sein.

Weiterhin kann vorgesehen sein, daß das Band in einer, vorzugsweise im Mittelabschnitt quergeteilten oder dort teilbaren Hülle angeordnet ist, die nach Implantation des Bandes über die Bandenden abziehbar ist. Die Hülle dient einerseits zum Schutz des Bandes insbesondere gegen Kontamination. Andererseits kann die Hülle auch die Einführbarkeit des Bandes erleichtern, wenn dieses eine rauhe Oberfläche besitzt. Das Band kann auch in Längsrichtung auftrennbar ausgebildet sein, beispielsweise durch eine entsprechende Überlappung. Ferner können den Bandenden noch Fixierungseinrichtungen zugeordnet sein, mit denen die Bandenden auf der Bauchdecke fixierbar sind, um beispielsweise beim Abziehen der Hülle zu vermeiden, daß die Bandenden nach innen rutschen.

Weitere Merkmale der Erfindung ergeben sich aus der nachfolgenden Beschreibung von Ausführungsformen der Erfindung zusammen mit der Zeichnung und den Unteransprüchen. Hierbei können die einzelnen Merkmale jeweils für sich alleine oder in Kombination miteinander ausgebildet sein.

In der Zeichnung zeigen:
- Figur 1:: eine perspektivische, Ansicht einer Ausführungsform eines Instruments zur Einführung eines Inkontinenzbandes mit einem gebogenen Schaft und einem Handhabungsgriff für den Schaft in voneinander getrenntem Zustand,
- Figur 2:: die Ausführungsform nach Figur 1 in einer anderen perspektivischen Ansicht,
- Figur 3:: die gleiche Anordnung von Schaft und Handhabungsgriff wie in Figur 1 im Schnitt,
- Figur 4:: einen Längsschnitt mit in den Griff eingeführtem aber noch nicht verriegeltem Schaft,
- Figur 5:: einen Längsschnitt durch Schaft und Griff im verriegelten Zustand,
- Figur 6:: eine perspektivische Ansicht von Schaft und Griff in verriegeltem Zustand,
- Figur 7:: eine Ansicht des Schaftes in der Krümmungsebene des Schaftes,
- Figur 8:: eine Teilansicht des Schaftes mit daran befestigtem Inkontinenzband,
- Figur 9:: ein Schaftende einer anderen Ausführungsform in vergrößertem Zustand, das alternativ am einen Griff oder an einem Inkontinenzband befestigbar ist oder auf das eine Spitze aufsteckbar ist.
- Figur 10:: eine Seitenansicht einer anderen Ausführungsform eines Instrumentes zur Einführung eines Inkontinenzbandes,
- Figur 11:: eine gegenüber Figur 10 um 90° gedrehte Ansicht dieser Ausführungsform,
- Figur 12:: eine Ausführungsform eines Inkontinenzbandes und
- Figur 13:: eine andere Ausführungsform eines Inkontinenzbandes.

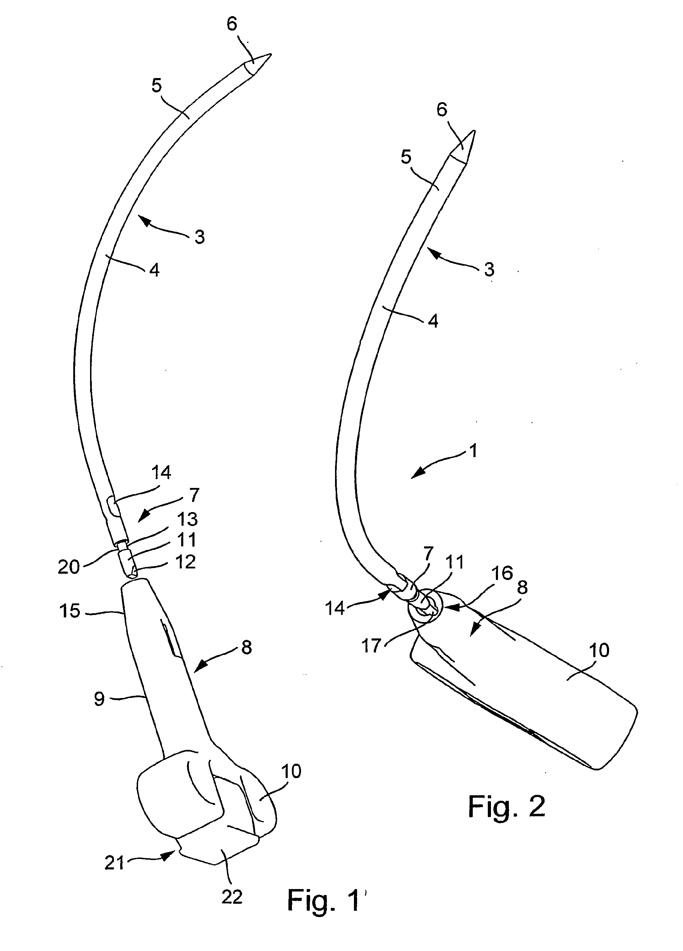

Bei der in den Figuren 1 bis 9 dargestellten Ausführungsform eines Platzierungsinstrumentes 1 für ein Inkontinenzband 2 ist ein Schaft zur Einführung des Inkontinenzbandes als gekrümmte massive Nadel 3 aus Edelstahl in Form eines gekrümmten Rundstabes 4 vorgesehen. Die Nadel besitzt an einem Einführende 5 eine kegelförmige Spitze 6. Diese kann auch als Schrägschliff derart ausgebildet sein, daß die Kante des Schliffes auf der Krümmungsinnenseite liegt. Die Nadelspitze ist nicht scharf, damit Verletzungen der Bauchorgane bei der Einführung des Inkontinenzbandes vermieden werden. Die Durchtrennung von Bauchwand und Vaginawand erfolgt ohnehin durch andere Instrumente, wie Skalpelle, so daß die Nadel im wesentlichen nur die Funktion eines Führungsorganes besitzt. Das der Spitze 6 gegenüber liegende Ende ist als Befestigungsende 7 ausgebildet und weist Einrichtungen zur alternativen Befestigung an einem Griff 8 oder an dem Inkontinenzband 2 auf.

Der Griff 8 besteht aus Kunststoff oder Stahl. Er weist einen im wesentlichen als Hohlzylinder ausgebildeten Längsabschnitt 9 auf, der sowohl zur Aufnahme und Befestigung des Nadelendes 7 als auch zum Umfassen mit der Hand dient. Am hinteren Griffende weist der Griff eine querverlaufende Verbreiterung 10 auf, die dem Griff im wesentlichen eine T-Form verleiht und ebenfalls zum Ergreifen dient, und eine feinfühlige Schwenkbewegung der Nadel ermöglicht.

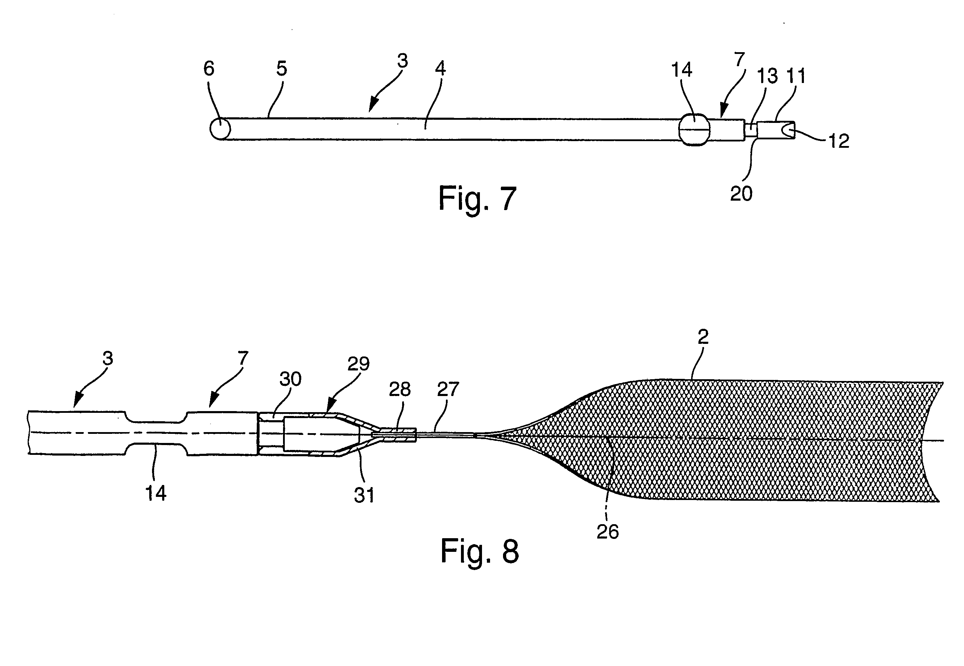

Wie aus Figur 7 ersichtlich, besitzt das Befestigungsende 7 der Nadel 3 einen zylindrischen Abschnitt 11 mit gegenüber dem normalen Durchmesser der Nadel verringertem Durchmesser, der zum freien Ende hin eine schräg verlaufende oder zwei schräg verlaufende gegenüber liegende Abflachungen 12 aufweist und gegen den Rundstab 4 der Nadel 3 durch eine Ringnut 13 abgesetzt ist, die einen zylindrischen Zwischenabschnitt mit noch geringerem Durchmesser bildet. Weiterhin ist das Nadelende im Bereich des vollen Durchmessers mit einer vom runden Querschnitt der Nadel abweichenden Verdrehsicherung insbesondere in Form einer Flachpressung 14 versehen.

Der Griff 8 ist zur Schnittebene in der T-Form symmetrisch ausgebildet und besitzt im Innern eine Aufnahme zur drehfesten Halterung der Nadel. Hierzu weist er an seinem konisch verjüngten Befestigungsende 15 eine Einschuböffnung 16 auf, deren Innendurchmesser dem normalen Außendurchmesser der Nadel entspricht und die noch zwei seitliche flache längsnutförmige Erweiterungen 17 besitzt, deren Größe der Flachpressung 14 der Nadel entspricht und den verdrehgesicherten Sitz der Nadel in richtiger Ausrichtung zum Griff gewährleistet. Bei dieser Ausrichtung steht die Krümmungsebene der Nadel senkrecht zur Ebene der T-Form des Griffes.

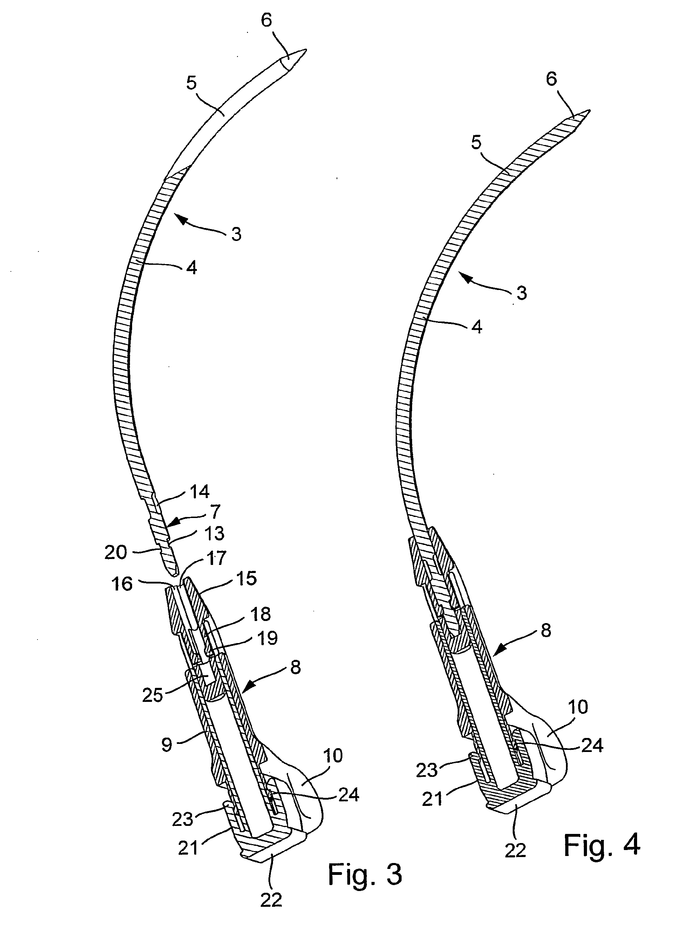

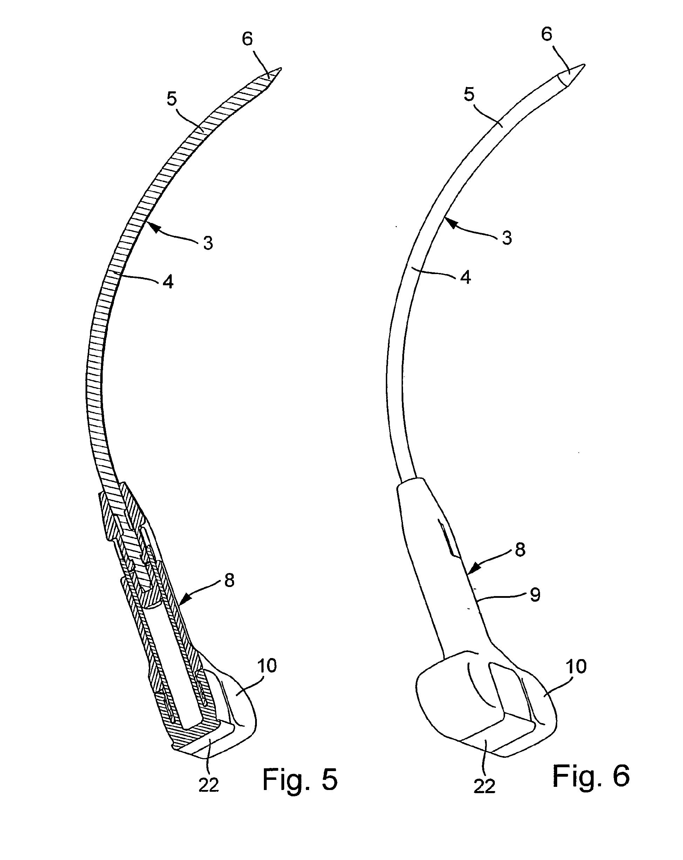

Die Griffwandung des hohlzylinderförmigen Längsabschnittes 9 besitzt zwei zur Ebene der T-Form spiegelbildlich gegenüber liegende fensterartige durch Ausparungen gebildete Materialschwächungen, die als Rastfedern 18 ausgebildet sind. Diese Rastfedern 18 sind längliche Materialfahnen, die an ihrem zur Einschuböffnung des Griffes weisenden Ende einstückig mit dem Material des Längsabschnittes 9 ausgebildet sind und an ihrem freien Ende nach innen weisende Rastnasen 19 besitzen. Die Federn 18 rasten, wie in Figur 4 dargestellt, mit ihren Nasen 19 in die Ringnut 13 des Nadelendes 7 ein, sind jedoch nicht selbstverriegelnd. Vielmehr besitzen die Rastnasen in und gegen die Einschubrichtung weisende Abflachungen und sind beim Einschieben des Nadelendes 7 durch die dazu ausgerichteten Abflachungen 12 des Nadelendes und beim Herausziehen durch die endseitige Ringschulter 20 der Ringnut 13 federnd auseinanderdrückbar. Die Verriegelung der Rastfedern erfolgt mit Hilfe eines im Griff angeordneten längs verschiebbaren hülsenförmigen Schiebers 21, der die Rastfedern 18 in Verriegelungsstellung von außen übergreift (Figur 5) und mit seinem freien zur Betätigung dienenden Ende 22 bündig in der Verbreiterung 10 am Griffende liegt und dort selbstfedernd am Gehäuse des Griffs einrastet. In der entriegelten Stellung (Figur 3 und 4) gibt der Schieber 21 die Rastfedern 18 frei und steht am Griffende über. Rastelemente 23 des Schiebers dienen in entriegelter Stellung zusammen mit entsprechenden Wirkelementen 24 des Griffes auch als Herausfallsicherung für den Schieber 21. Zur Reinigung ist der Griff zerlegbar, d.h. der Schieber 21 kann zur Entfernung über die Wirkelemente 24 des Griffes 8 herausgezogen werden. Im Innern besitzt der Griff auch noch einen Öffnungsabschhitt 25 mit verringertem Innendurchmesser, der dem Außendurchmesser des zylindrischen Abschnittes 11 des Nadelendes 7 entspricht und zur Aufnahme dieses Abschnittes dient. Dadurch wird ein wackelfreier Sitz der Nadel im Griff gewährleistet.

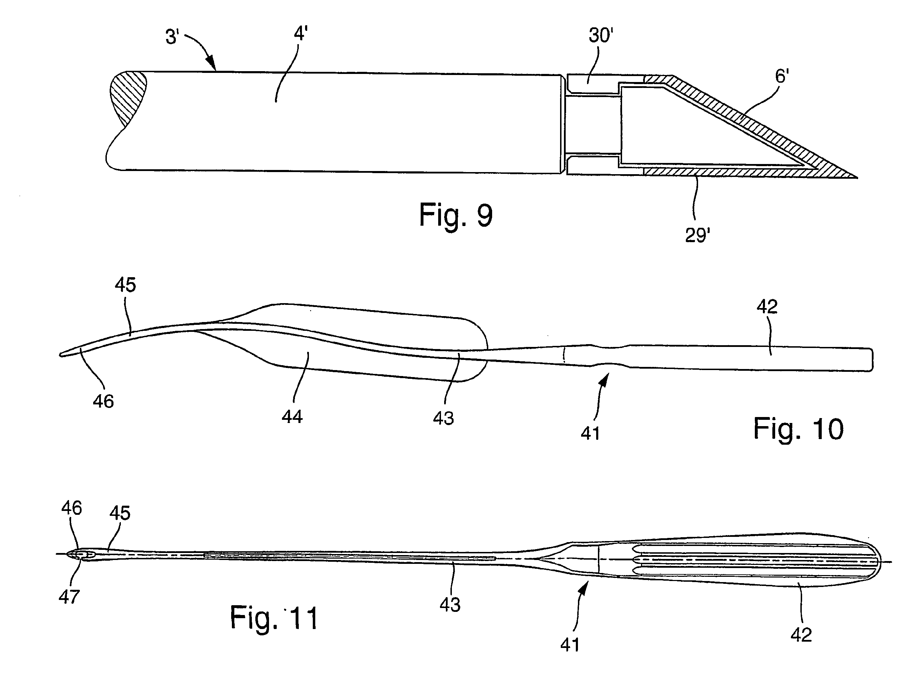

Das Band 2 wird von einem flachen Streifen aus einem textilen Netzmaterial gebildet. Beide Bandenden sind entlang der Mittellinie 26 des Bandes gefaltet, so daß sich ein Endabschnitt 27 mit halber Breite ergibt, dessen Ebene senkrecht zur Bandebene liegt. Diese Endabschnitte 27 sind in ein flachgepresstes Ende 28 einer Befestigungshülse 29 fest eingeklemmt, die als Aufschiebhülse auf das Nadelende 7 zum einrastenden Überschieben des zylindrischen Abschnittes 11 mit verringertem Durchmesser ausgebildet ist. Die Befestigungshülse hat den gleichen Außendurchmesser wie der Rundstab 4 der Nadel. Zur Befestigung am Nadelende 7 ist das freie Ende 30 der Befestigungshülse 29 längs geschlitzt, wobei die Ränder der dadurch gebildeten Fahnen nach innen abgekantet oder verdickt ausgebildet sind und in die Längsnut 13 des Nadelendes einrasten und die Ringschulter 20 formschlüssig hintergreifen. Das Band 2 ist an der Nadel 3 verdrehsicher befestigbar, insbesondere in einer Lage, bei der die Bandebene in der Ebene der Nadelkrümmung liegt. Hierzu wirken die eine (Figur 9) oder beide Abflachungen 12 des zylindrischen Abschitts 11 mit durch die Flachpressung der Befestigungshülse 29 gebildeten Keilflächen 31 zusammen. Auch eine andere Ausbildung der Verdrehsicherung ist möglich.

Bei der dargestellten Ausführungsform der Erfindung kann durch die Bandbefestigung verhindert werden, daß die beiden Enden des Bandes seitlich nacheinander an nur einer Nadel befestigt werden, d.h. es kann verhindert werden, daß dieselbe Nadel bei der Operation zweimal verwendet wird. Hierzu ist vorgesehen, daß die Verbindung der Befestigungshülse 29 am Nadelende 7 unlösbar ist oder nur mit einem hierzu ausgebildeten Werkzeug gelöst werden kann. Dadurch wird die Befestigung des anderen Bandendes am Nadelende 7 unmöglich gemacht und die Wiederverwendung der Nadel bei derselben Operation verhindert. Im Falle einer unlösbaren Verbindung wird die Nadel 3 nach dem Abschneiden des Bandes 2 zusammen mit der Befestigungshülse 29 verworfen.

Es kann weiterhin vorgesehen sein, dass die Nadel 3' an beiden Enden Spitzen 6' aufweist, in dem beispielsweise auch das Befestigungsende 7 spitz ausgebildet oder schräg angeschliffen ist. Alternativ kann auch vorgesehen sein, dass Nadelspitzen an der Nadel 3', insbesondere an dem Rundstab 4' befestigbar ausgebildet sind, wie dies in Figur 9 dargestellt ist. Beide Nadelenden können für eine solche Befestigung einer Spitze 6' vorgesehen sein. So können beide Nadelenden wie das Befestigungsende 7' ausgebildet sein. Statt einer Befestigungshülse ist dann eine eine Spitze 6' aufweisende Hülse 29' aufsteckbar. Es kann auch vorgesehen sein, daß die Spitzen wiederabnehmbar ausgebildet sind und nach Abnehmen der Spitze die am Band befestigte Befestigungshülse aufgesteckt wird.

Die in den Figuren 1 bis 9 dargestellten Instrumente sind aufgrund ihrer Variationsmöglichkeiten vielseitig verwendbar und können sowohl für Operationen eingesetzt werden, bei denen der Schaft durch die Bauchwand hindurch von oben nach unten geführt wird oder von unten ausgehend, bei weiblichen Patienten beispielsweise durch die Vaginalwand, nach oben bis zur Bauchdecke. Das Band kann, wie beim Nähen, von der Nadel durchgezogen werden, indem die Nadel durch den Unterleib hindurchgeführt wird und das Band nach sich zieht. Es ist auch möglich, das Band abzuholen, indem beispielsweise die Nadel von der Bauchwand aus nach unten geführt wird, das Band dann an der zugänglichen Nadelspitze befestigt und beim Zurückziehen der Nadel mitgezogen wird.

Demgegenüber besitzt die in den Figuren 10 und 11 dargestellte Ausführungsform eines Instrumentes zum Platzieren eines Inkontinenzbandes weniger Anwendungsmöglichkeiten. Das dort abgebildete Instrument 41 ist jedoch einfacher aufgebaut und weist einige Besonderheiten auf. Das chirurgische Instrument 41 besitzt einen Griff 42 und einen Schaft 43, die einstückig miteinander ausgebildet sind und zweckmäßigerweise aus Edelstahl bestehen. Der Griff 42 ist flach und langgestreckt und kann auch eine andere für chirurgische Instrumente geeignete Form besitzen. Der Schaft 43 verläuft in einer sehr flachen S-Kurve. Er besitzt einen sich im wesentlichen an den Griff 42 anschließenden verbreiterten Abschnitt 44 in Form eines Flachstabes mit parallelen stumpfen Längskanten und einen sich daran anschließenden gekrümmten Abschnitt 45 mit im wesentlichen kreisförmigem Querschnitt, der an seiner Spitze 46 eine seitlich offene Fangnadel 47 zur Befestigung eines Inkontinenzbandes besitzt. Der Übergang vom breiten Abschnitt 44 in den dünnen Abschnitt 45 erfolgt allmählich unter Verjüngung des breiten Abschnittes. Die Ebene des breiten Abschnittes liegt in der Krümmungsebene des gekrümmten Abschnittes 45. Im Vergleich zum Griff 42 verlaufen die Längskanten des verbreiterten Abschnittes 44 leicht nach oben und der sich daran anschließende gekrümmte Abschnitt 45 leicht nach unten. Der Schaft kann aber auch als gekrümmter Stab mit im wesentlichen kreisförmigem Querschnitt ausgebildet sein.

Die Ausführungsform nach Figur 10 ist dazu vorgesehen, daß der Schaft durch die Bauchdecke hindurch in den Bauchraum nach unten geführt wird. Dabei hat der breite Abschnitt 44 die Aufgabe, dem Stichkanal durch die Bauchdecke und das darunter liegende Binde- und Fettgewebe bis zur Vaginalwand eine verbreiterte Form zu verleihen, die in etwa der Form des Inkontinenzbandes entspricht. Die Verbreiterung kann sich im wesentlichen über die gesamte Länge des Schaftes erstrecken. Bei in den Bauchraum eingeführtem Schaft kann das Band an der unten, beispielsweise durch die Vaginalwand, hindurchgeführten Spitze 46 des Schaftes 43 befestigt werden und beim Zurückziehen des Instrumentes mitgezogen werden. Bei zwei Einstichen in die Bauchdecke rechts und links oder vorzugsweise einem zentralen Einstich oberhalb des Schambeines können somit beide Bandenden rechts und links von der Harnröhre hochgezogen werden, so daß sich eine die Harnröhre untergreifende U-förmige Bandführung ergibt.

Die Schaftspitze kann für eine Befestigung mit Sicherungseinrichtung ausgebildet sein, die ein unbeabsichtigtes Lösen des Bandes vom Schaft verhindert. Bei der dargestellten Ausführungsform ist die Schaftspitze 46 vorzugsweise als sogenannte Reverdinnadel ausgebildet. Diese besitzt eine Öse, die öffenbar und verschließbar ist und im geöffneten Zustand hakenförmig ausgebildet ist. Es ist aber auch möglich, die Befestigungseinrichtung an der Spitze 46 anders zu gestalten, beispielsweise als einfache Öse oder indem die Spitze als abnehmbare Kappe ausgebildet ist und nach Abnehmen der Spitze ein Kupplungsteil freigelegt wird, das mit einem zusammenwirkenden Kupplungsteil des Bandendes verbindbar ist, wie dies im Zusammenhang mit Figur 9 beschrieben wird. Die Öse kann auch durch eine Überwurfmutter verschließbar sein.

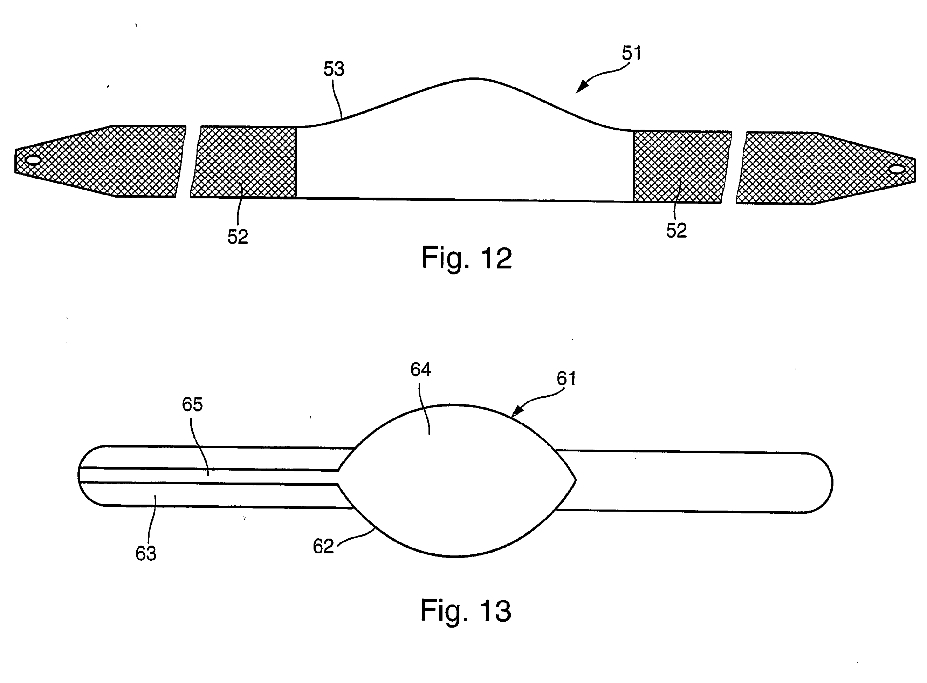

Bei der Ausführungsform eines Inkontinenzbandes nach Figur 12 weist das Inkontinenzbandes 51 voneinander verschiedene Längsabschnitte auf. Zwei Endabschnitte 52 von je etwa 10 bis 20 cm Länge und etwa 2 cm Breite sind als grobmaschige textile Bänder ausgebildet, die an ihren Enden abgerundet sind oder sich V-förmig verjüngen und Löcher oder sonstige Befestigungseinrichtungen zur Befestigung an einem Einführinstrument aufweisen. Ein Mittelabschnitt 53 besteht aus resorbierbarem Kunststoff und ist im Vergleich zu den Endabschnitten 52 auf einer Seite nach Art einer Auswölbung verbreitert. Der Abschnitt besteht aus glattem Folienmaterial und besitzt glatte Ränder. Der Abschnitt ist zum Unterlegen der Harnröhre bestimmt. Durch die glatte Ausbildung und die die Harnröhre schonende Gestaltung wird eine Schädigung der Harnröhre durch unerwünschte Wucherung von neugebildetem Bindegewebe vermieden. Die Einbringung dieses Bandabschnittes als Fremdkörper sorgt für eine ausreichende Gewebeneubildung, die die Harnröhre von unten her unterstützt. Da damit die Funktion des Implantats erfüllt ist, kann insbesondere dieser Bandabschnitt resorbierbar gestaltet sein.

Die Degradationszeit und Resorptionsdauer des Materials des Mittelabschnittes kann in bekannter Weise durch entsprechende Copolymerisation vorbestimmt werden. Als Polymere kommen synthetische Polymere in Frage, insbesondere solche, die durch Polymerisation bzw. Mischpolymerisation von Lactid, Glykolid, Trimethylencarbonat, Dioxanon und ε-Caprolacton erhältlich sind.

Bei der in Figur 13 dargestellten Ausführungsform ist ein Inkontinenzband vorgesehen, das zum dauernden Verbleib im Körper bestimmt ist. Das Inkontinenzband 61 ist wiederum in verschiedene Abschnitte unterteilt, wobei mindestens ein mittlerer Abschnitt 62 als mit einem Fluid, beispielsweise Wasser oder flüssigem Kontrastmittel, füllbare Kammer ausgebildet ist. Hierzu kann der mittlere Abschnitt 62 oder auch das gesamte Band schlauchförmig ausgebildet sein. Hinsichtlich Aufbau und Material gibt es verschiedene Möglichkeiten. Bei der in Figur 13 dargestellten Ausführungsform besteht das Band mindestens im mittleren Abschnitt und mindestens in einem sich daran anschließenden Kanalabschnitt 63 aus thermoplastischen Material. Mit Hilfe von Schweißnähten läßt sich unter Verbindung der beiden Wandungen des Schlauches die Kammer 64 und ein mit dieser kommunizierender Fluidkanal 65 ausbilden, der entlang des Kanalabschnittes 63 verläuft und durch den die Kammer befüllbar ist und durch den das Füllvolumen bei Bedarf nachträglich korrigiert werden kann. Der Fluidkanal 65 kann nach Befüllung beispielsweise durch Verschweißen verschlosssen werden. Er kann unter der Bauchhaut auch durch ein von außen zugängliches Ventil, beispielsweise durch eine durchstechbare Membran zugänglich sein.

Es ist alternativ auch möglich, zumindest die Kammer aus einem Elastomer auszubilden, beispielsweise Silikon, das durchstechbar und selbstabdichtend ist. Dann kann ein besonderer Fluidkanal entfallen. Weiterhin ist es möglich, das ganze Band schlauchförmig auszubilden und lediglich die Schlauchenden zu verschließen. Wesentlich ist, daß auch das Band mit befüllbarer Kammer bandförmig ausgebildet ist, wodurch die minimalinvasive Platzierung des Bandes ermöglicht wird, ohne das eine Eröffnung des Bauchraumes erforderlich ist.

## Patentansprüche

1. Chirurgisches Instrument (1) zum Platzieren eines Haminkontinenzbandes (2) im Unterleib von Patienten, insbesondere weiblichen Patienten, mit mindestens einem gebogenen Schaft (3) zum Durchdringen des Unterleibes, einem dem Schaft (3) zugeordneten Handgriff (8) für den Schaft und mindestens einer Befestigungseinrichtung (7; 47) für das Band am Schaft, **dadurch gekennzeichnet, dass** das Bandende (27) mittels der Befestigungseinrichtung (7; 47) mit dem Schaft (3) unlösbar zu verbinden ist, wobei das Bandende (27) beim Trennen des Bandes (2) vom Schaft an diesem untrennbar verbleibt.

2. Chirurgisches Instrument nach Anspruch 1, **dadurch gekennzeichnet, dass** der Schaft (3) zur lösbaren Verbindung mit dem Griff (8) ausgebildet und insbesondere axial und drehgesichert mit dem Griff (8) verbindbar ist.

3. Chirurgisches Instrument nach Anspruch 1 oder 2, **dadurch gekennzeichnet, dass** der Schaft (3) derartig, insbesondere asymmetrisch, gekrümmt ist, dass er alternativ von der Bauchhöhle aus oder vom Schritt aus in den Unterleib einführbar ist.

4. Chirurgisches Instrument nach einem der vorhergehenden Ansprüche, **dadurch gekennzeichnet, dass** der Schaft an beiden Enden Einführspitzen zum Durchdringen des Unterleibes aufweist oder mit solchen verbindbar ist.

5. Chirurgisches Instrument nach einem der vorhergehenden Ansprüche, **dadurch gekennzeichnet, dass** der Schaft an beiden Enden zur alternativen lösbaren Verbindung mit dem Griff ausgebildet ist.

6. Chirurgisches Instrument nach einem der vorhergehenden Ansprüche, **dadurch gekennzeichnet, dass** der Schaft an beiden Enden zur alternativen Verbindung mit dem Band ausgebildet ist.

7. Chirurgisches Instrument nach einem der vorhergehenden Ansprüche, **dadurch gekennzeichnet, dass** das Band (2) nur bei entferntem Griff (8) vom Schaft (3) an dessen entsprechendem Ende befestigbar ist.

8. Chirurgisches Instrument nach einem der vorhergehenden Ansprüche, **dadurch gekennzeichnet, dass** der Schaft (3) zur Einmalverwendung vorgesehen ist, jedem Bandende (27) ein eigener Schaft (3) zugeordnet ist, und der Schaft (3) eine Sicherungseinrichtung (7; 29) zur Verhinderung der Wiederverwendung aufweist.

9. Chirurgisches Instrument nach einem der vorhergehenden Ansprüche, **dadurch gekennzeichnet, dass** das Band (2) mittels einem am Bandende (27) befestigten Kupplungsstück (29) am Schaft (3) befestigbar ist und das Kupplungsstück (29) beim Trennen des Bandes vom Schaft an diesem verbleibt.

10. Chirurgisches Instrument nach Anspruch 9, **dadurch gekennzeichnet, dass** das Schaftende (7) und das Kupplungsstück (29) als Steckverbindung ausgebildet sind.

11. Chirurgisches Instrument nach einem der vorhergehenden Ansprüche, **dadurch gekennzeichnet, dass** das Band (2) mit Hilfe einer selbstsperrenden Verbindung (7; 20; 29; 30) am Schaft (3) befestigbar ist.

12. Chirurgisches Instrument nach einem der vorhergehenden Ansprüche, **dadurch gekennzeichnet, dass** der Schaft (3) in den Griff (8) einsteckbar ausgebildet ist und der Griff (8) zur Axialverriegelung in Ausnehmungen (13) des Schaftes (3) vorzugsweise federnd eingreifbare Rastglieder (18) aufweist, die in Raststellung verriegelbar sind.

13. Chirurgisches Instrument nach Anspruch 12, **dadurch gekennzeichnet, dass** die Rastglieder (18) mittels eines Sperrschiebers (21) des Griffs (8) verriegelbar sind, der vorzugsweise zumindest in Schließstellung kraftschlüssig gehalten ist.

14. Chirurgisches Instrument nach einem der vorhergehenden Ansprüche, **dadurch gekennzeichnet, dass** der Querschnitt des Schaftes (43) in einem Bereich, der außerhalb des vom Handgriff (42) umfassten Bereiches liegt, von der Kreisform abweicht, insbesondere in einer Richtung, die vorzugsweise in der Krümmungsebene des Schaftes liegt, um ein mehrfaches größer ist als in der dazu senkrechten Richtung.

15. Chirurgisches Instrument nach einem der vorhergehenden Ansprüche, **dadurch gekennzeichnet, dass** der Schaft mindestens über einen Teil seiner Länge, insbesondere über seine gesamte Länge, als flacher, insbesondere gekrümmter Stab ausgebildet ist.

16. Chirurgisches Instrument nach einem der vorhergehenden Ansprüche, **dadurch gekennzeichnet, dass** der Schaft mindestens in einem Längsabschnitt (44) eine Breite besitzt, die in etwa der Breite des daran zu befestigenden Bandes entspricht.

17. Chirurgisches Instrument nach einem der vorhergehenden Ansprüche, **dadurch gekennzeichnet, dass** der Schaft in einem Längsabschnitt eine Breite besitzt, die größer als die des Bandes ist.

18. Chirurgisches Instrument nach Anspruch 17, **dadurch gekennzeichnet, dass** Änderungen in der Breite vorzugsweise allmähliche Übergänge besitzen.

19. Chirurgisches Instrument nach einem der vorhergehenden Ansprüche, **dadurch gekennzeichnet, dass** das Band (2) am Schaft (3) verdrehsicher fixierbar ist, insbesondere in einer Ebene, die der Biegungsebene und/oder der Ebene der Flachseite des Schaftes entspricht.

20. Chirurgisches Instrument nach einem der vorhergehenden Ansprüche, **dadurch gekennzeichnet, dass** es, insbesondere der Griff (8), mindestens teilweise aus Kunststoff besteht.

21. Chirurgisches Instrument nach einem der Ansprüche 1, 8 bis 11 und 14 bis 19, **dadurch gekenntzeichnet, dass** der Schaft (43) und Griff (42) unlösbar miteinander verbunden, insbesondere einstückig ausgebildet sind und am freien Schaftende (46) die Befestigungseinrichtung (47) für das Band vorgesehen ist.

## Claims

1. Surgical instrument (1) for placing a urinary incontinence tape (2) in the lower abdomen of patients, especially of female patients, with at least one curved shaft (3) for penetrating the lower abdomen, a handle (8) for the shaft assigned to the shaft (3), and at least one securing device (7; 47) for securing the tape on the shaft, **characterized in that** the tape end (27) is to be connected non-releasably to the shaft (3) by means of the securing device (7; 47), with the tape end (27) remaining inseparably on the shaft when the tape (2) is separated from the latter.

2. Surgical instrument according to Claim 1, **characterized in that** the shaft (3) is designed for releasable connection to the handle (8) and can in particular be connected to the handle (8) axially and secure against rotation.

3. Surgical instrument according to Claim 1 or 2, **characterized in that** the shaft (3) is curved, in particular asymmetrically curved, in such a way that it can be inserted into the lower abdomen either from the abdominal cavity or from the crotch.

4. Surgical instrument according to one of the preceding claims, **characterized in that**, at both ends, the shaft has insertion tips for penetrating the lower abdomen or can be connected to such insertion tips.

5. Surgical instrument according to one of the preceding claims, **characterized in that** the shaft is designed at both ends for alternative releasable connection to the handle.

6. Surgical instrument according to one of the preceding claims, **characterized in that** the shaft is designed at both ends for alternative connection to the tape.

7. Surgical instrument according to one of the preceding claims, **characterized in that** the tape (2) can be secured on the corresponding end of the shaft (3) only when the handle (8) is removed from the shaft (3).

8. Surgical instrument according to one of the preceding claims, **characterized in that** the shaft (3) is intended to be used just once, each tape end (27) being assigned its own shaft (3), and the shaft (3) has a safety device (7; 29) for avoiding reuse.

9. Surgical instrument according to one of the preceding claims, **characterized in that** the tape (2) can be secured on the shaft (3) by means of a coupling piece (29) secured on the tape end (27), and the coupling piece (29) remains on the shaft when the tape is separated from the shaft.

10. Surgical instrument according to Claim 9, **characterized in that** the shaft end (7) and the coupling piece (29) are designed as a plug-and-socket connection.

11. Surgical instrument according to one of the preceding claims, **characterized in that** the tape (2) can be secured on the shaft (3) with the aid of a self-locking connection (7; 20; 29; 30).

12. Surgical instrument according to one of the preceding claims, **characterized in that** the shaft (3) is designed to be inserted into the handle (8) and, in order to permit axial locking, the handle (8) has detents (18) which are able to engage preferably resiliently in recesses (13) of the shaft (3) and which can be locked in the detent position.

13. Surgical instrument according to Claim 12, **characterized in that** the detents (18) can be locked by means of a blocking slide (21) of the handle (8), which blocking slide (21) is preferably held with a force fit at least in the closed position.

14. Surgical instrument according to one of the preceding claims, **characterized in that** the cross section of the shaft (43), in an area lying outside the area covered by the handle (42), deviates from the circular shape, and in particular in a direction lying preferably in the plane of curvature of the shaft is several times greater than in the direction perpendicular thereto.

15. Surgical instrument according to one of the preceding claims, **characterized in that** the shaft is designed as a flat, in particular curved rod, at least along part of its length, in particular along its entire length.

16. Surgical instrument according to one of the preceding claims, **characterized in that** the shaft, at least in one section (44) of its length, has a width that corresponds approximately to the width of the tape to be secured thereon.

17. Surgical instrument according to one of the preceding claims, **characterized in that** the shaft, in one section of its length, has a width that is greater than the width of the tape.

18. Surgical instrument according to Claim 17, **characterized in that** changes in the width are preferably gradual transitions.

19. Surgical instrument according to one of the preceding claims, **characterized in that** the tape (2) can be fixed on the shaft (3) in a manner secure against twisting, particularly in a plane corresponding to the plane of flexure and/or the plane of the flat side of the shaft.

20. Surgical instrument according to one of the preceding claims, **characterized in that** the instrument, in particular the handle (8), is made at least partly of plastic.

21. Surgical instrument according to one of Claims 1, 8 to 11 and 14 to 19, **characterized in that** the shaft (43) and the handle (42) are connected non-releasably to each other, in particular are formed in one piece, and the securing device (47) for the tape is provided at the free end (46) of the shaft.

## Revendications

1. Instrument chirurgical (1) destiné à placer une bandelette d'incontinence urinaire (2) dans le bas-ventre de patients, en particulier de patients féminins, comprenant au moins une tige (3) incurvée pour pénétrer dans le bas-ventre, une poignée (8) attribuée à la tige (3) pour la tige et au moins un dispositif de fixation (7 ; 47) pour la bandelette sur la tige, **caractérisé en ce que** l'extrémité de la bandelette (27) doit être reliée de manière inséparable à la tige (3) au moyen du dispositif de fixation (7 ; 47), l'extrémité de la bandelette (27), lors de la séparation de la bandelette (2) d'avec la tige, restant sur celle-ci sans pouvoir être séparée.

2. Instrument chirurgical selon la revendication 1, **caractérisé en ce que** la tige (3) est formée pour être reliée de manière séparable à la poignée (8) et peut être en particulier reliée à la poignée (8) de manière axiale et en empêchant la rotation.

3. Instrument chirurgical selon la revendication 1 ou 2, **caractérisé en ce que** la tige (3) est incurvée, en particulier de manière asymétrique, de telle sorte qu'elle puisse être introduite dans le bas-ventre en variante depuis la cavité abdominale ou depuis l'entrejambe.

4. Instrument chirurgical selon l'une des revendications précédentes, **caractérisé en ce que** la tige présente sur les deux extrémités des pointes d'introduction pour pénétrer dans le bas-ventre ou peut être reliée à de telles pointes.

5. Instrument chirurgical selon l'une des revendications précédentes, **caractérisé en ce que** la tige est formée sur les deux extrémités pour être reliée manière séparable en variante avec la poignée.

6. Instrument chirurgical selon l'une des revendications précédentes, **caractérisé en ce que** la tige est formée sur les deux extrémités pour être reliée en variante avec la bandelette.

7. Instrument chirurgical selon l'une des revendications précédentes, **caractérisé en ce que** la bandelette (2) ne peut être fixée à la tige (3) sur son extrémité correspondante que lorsque la poignée (8) est éloignée.

8. Instrument chirurgical selon l'une des revendications précédentes, **caractérisé en ce que** la tige (3) est prévue pour un usage unique, chaque extrémité de la bandelette (27) est attribuée sa propre tige (3) et la tige (3) présente un dispositif de sécurité (7 ; 29) pour empêcher une réutilisation.

9. Instrument chirurgical selon l'une des revendications précédentes, **caractérisé en ce que** la bandelette (2) peut être fixée sur la tige (3) au moyen d'une pièce de couplage (29) fixée sur l'extrémité de la bandelette (27) et la pièce de couplage (29) reste sur la tige lors de la séparation de la bandelette d'avec celle-ci.

10. Instrument chirurgical selon la revendication 9, **caractérisé en ce que** l'extrémité de la tige (7) et la pièce de couplage (29) sont formées comme une liaison par emboîtement.

11. Instrument chirurgical selon l'une des revendications précédentes, **caractérisé en ce que** la bandelette (2) peut être fixée sur la tige (3) au moyen d'une liaison autobloquante (7 ; 20 ; 29 ; 30).

12. Instrument chirurgical selon l'une des revendications précédentes, **caractérisé en ce que** la tige (3) est formée en étant insérable dans la poignée (8) et la poignée (8) présente des éléments d'encliquetage (18) pouvant de préférence se mettre en prise de manière élastique dans des évidements (13) de la tige (3) pour un verrouillage axial, lesquels sont verrouillables en position d'encliquetage.

13. Instrument chirurgical selon la revendication 12, **caractérisé en ce que** les éléments d'encliquetage (18) sont verrouillables au moyen d'un curseur de blocage (21) de la poignée (8) qui est maintenu de préférence par adhérence des forces au moins en position fermée.

14. Instrument chirurgical selon l'une des revendications précédentes, **caractérisé en ce que** la section transversale de la tige (43) dévie de la forme circulaire dans une zone à l'extérieur de la zone delimitée par la poignée (42), en particulier dans une direction qui se situe de préférence dans le plan de courbure de la tige, est plusieures fois plus grande que dans la direction verticale à celle-ci.

15. Instrument chirurgical selon l'une des revendications précédentes, **caractérisé en ce que** la tige est formée sur au moins une partie de sa longueur, en particulier sur toute sa longueur, comme un bâtonnet plat, en particulier incurvé.

16. Instrument chirurgical selon l'une des revendications précédentes, **caractérisé en ce que** la tige possède au moins dans une section longitudinale (44) une largeur qui correspond à environ la largeur de la bandelette à fixer dessus.

17. Instrument chirurgical selon l'une des revendications précédentes, **caractérisé en ce que** la tige possède dans une section longitudinale une largeur qui est supérieure à celle de la bandelette.

18. Instrument chirurgical selon la revendication 17, **caractérisé en ce que** des modifications de la largeur possèdent de préférence des jonctions graduelles.

19. Instrument chirurgical selon l'une des revendications précédentes, **caractérisé en ce que** la bandelette (2) peut être fixée de manière à ne pas pouvoir tourner sur la tige (3), en particulier dans un plan qui correspond au plan de flexion et/ou au plan du côté plat de la tige.

20. Instrument chirurgical selon l'une des revendications précédentes, **caractérisé en ce qu'**il est composé, en particulier la poignée (8) est composée, au moins partiellement de plastique.

21. Instrument chirurgical selon l'une des revendications 1, 8 à 11 et 14 à 19, **caractérisé en ce que** la tige (43) et la poignée (42) sont reliées entre elles de manière inséparable, en particulier sont formées de manière monobloc et le dispositif de fixation (47) de la bandelette est prévu sur l'extrémité libre de la tige (46).
